# EUROPÄISCHE PATENTSCHRIFT

(11) **EP 3 045 435 B1**
(45) Veröffentlichungstag und Bekanntmachung des Hinweises auf die Patenterteilung: **24.10.2018**
(21) Anmeldenummer: 16159020.3
(22) Anmeldetag: 18.04.2011
(51) Int. Cl.: C03C 10/12, A61K 6/027, C03C 3/097

(54) **LITHIUMSILIKAT-GLASKERAMIK UND -GLAS MIT GEHALT AN ZRO2**
LITHIUM SILICATE GLASS CERAMIC AND GLASS WITH ZRO2 CONTENT
VITROCERAMIQUE ET VERRE AU LITHIUM-SILICE AYANT UNE TENEUR EN ZRO2

(30) Priorität: 16.04.2010 EP 10160222
(43) Veröffentlichungstag der Anmeldung: 20.07.2016
(62) Teilanmeldung aus: 11162840.0
(73) Patentinhaber: Ivoclar Vivadent AG, 9494 Schaan (LI)
(72) Erfinder: Ritzberger, Christian, 9472 Grabs (CH); Dellagiacoma, Ricardo, 6800 Feldkirch (AT); Schweiger, Marcel, 7000 Chur (CH); Bürke, Harald, 6820 Frastanz (AT); Höland, Wolfram, 9494 Schaan (LI); Rheinberger, Volker, 9490 Vaduz (LI)
(74) Vertreter: Uexküll & Stolberg

(56) Entgegenhaltungen:
- EP-A1- 0 690 031
- WO-A1-2011/076422
- DE-A1-102005 028 637

## Beschreibung

Die Erfindung betrifft Lithiumsilikat-Glaskeramik, die ZrO₂ enthält und sich insbesondere zum Beschichten von Zirkonoxidkeramik eignet.

Zirkonoxidkeramiken zeichnen sich durch ausgezeichnete Biokompatibilität und hervorragende mechanische Eigenschaften aus, weshalb sie in den vergangenen Jahren in zunehmenden Umfang als Werkstoff für Implantate und Prothesen, aber auch als Gerüstwerkstoffe für dentale Restaurationen eingesetzt worden sind. Dabei werden vornehmlich Keramiken auf Basis von teilstabilisiertem Zirkonoxid verwendet.

In vielen Fällen ist es wünschenswert, die Oberfläche der Zirkonoxidkeramik durch Beschichtung mit einem anderen Material zu verändern. Gerade bei der Herstellung von dentalen Restaurationen auf Basis von Zirkonoxidkeramik wird eine solche Beschichtung regelmäßig verwendet, um der Restauration die gewünschten optischen Eigenschaften zu verleihen.

Zur Beschichtung oder Verblendung von Oxidkeramiken, wie Zirkonoxidkeramiken, sind in der Vergangenheit bereits Glaskeramiken eingesetzt worden. Dazu zählen auf Feldspat basierende Keramiken oder Fluoroapatit-Glaskeramiken.

Weiter sind Lithiumdisilikat-Glaskeramiken bekannt, die aufgrund ihrer hohen Transluzenz und sehr guten mechanischen Eigenschaften besonders im Dentalbereich und dabei vornehmlich zur Herstellung von Dentalkronen und kleinen Brücken Anwendung finden.

Die EP 1 505 041 beschreibt Lithiumsilikat-Glaskeramiken, die zusätzlich 0 bis 2 Gew.-% ZrO₂ enthalten können. Diese werden insbesondere in Form der Lithiummetasilikat-Glaskeramiken mittels CAD/CAM-Verfahren zu den gewünschten Dentalrestaurationen verarbeitet, wobei eine anschließende Wärmebehandlung die Umwandlung der Metasilikat-Phase in die hochfeste Disilikat-Phase bewirkt. Die Glaskeramiken können auch zum Überpressen von keramischen Restaurationen benutzt werden.

Die EP 1 688 398 beschreibt ähnliche Lithiumsilikat-Glaskeramiken, die im Wesentlichen frei von ZnO sind und neben anderen Komponenten 0 bis 4 Gew.-% ZrO₂ enthalten können. Für die Erzielung hoher Festigkeiten sind allerdings geringe Mengen von 0 bis 2 Gew.-% ZrO₂ bevorzugt. Auch diese Glaskeramiken dienen insbesondere zur Herstellung dentaler Restaurationen nach mechanischer Verarbeitung mittels CAD/CAM.

Diese aus dem Stand der Technik bekannten Lithiumsilikat-Glaskeramiken besitzen allerdings den Nachteil, dass sie nicht zum Beschichten von Zirkonoxidkeramik insbesondere mittels eines Aufpressvorgangs im viskosen Zustand geeignet sind. Denn nach dem Aufpressen durch den viskosen Fließvorgang entstehen Risse und Sprünge in der Glaskeramik. Somit hat ein derartiger Verbund nicht die mechanischen Eigenschaften, die gerade für den Einsatz als dentales Restaurationsmaterial unabdingbar sind.

Weiter sind aus der WO 2008/106958 Glaskeramiken mit Lithiumdisilikat als Hauptkristallphase bekannt, die zum Verblenden von dentalen Restaurationen aus Yttrium stabilisiertem Zirkoniumdioxid geeignet sein sollen. Allerdings enthalten diese Glaskeramiken nur Mengen von bis zu 6,0 Gew.-% an ZrO₂ und substantielle Mengen an Na₂O. Das vorhandene ZrO₂ dient dabei lediglich als klassischer Keimbildner zusammen mit optional vorhandenem weiteren Keimbildner wie TiO₂, um die Bildung der gewünschten Lithiumdisilikat-Kristallphase herbeizuführen. Die EP 0 690 031 offenbart Glaskeramiken mit 15-28 Gew.-% ZrO₂.

Ausgehend von den oben beschriebenen Nachteilen der bereits bekannten Glaskeramiken liegt der Erfindung die Aufgabe zugrunde, eine Glaskeramik zur Verfügung zu stellen, die insbesondere durch Aufpressen im viskosen Zustand auf eine Zirkonoxidkeramik geschichtet werden kann und dabei eine im wesentlichen von Rissen und Sprüngen freie Beschichtung bildet. Überdies soll die Glaskeramik in der Lage sein, einen festen Verbund mit der zu beschichtenden Zirkonoxidkeramik auszubilden, und sie soll über optische und mechanische Eigenschaften verfügen, um insbesondere als Beschichtungsmaterial für dentale Restaurationen aber auch als Werkstoff für dentale Restaurationen eingesetzt werden zu können.

Diese Aufgabe wird durch die Lithiumsilikat-Glaskeramik nach einem der Ansprüche 1 bis 9 gelöst. Gegenstand der Erfindung sind ebenfalls das Verfahren zur Herstellung der Glaskeramik nach Anspruch 10, die Verwendung nach den Ansprüchen 11 und 12, die dentale Restauration nach Anspruch 13 und die beschichtete Zirkonoxidkeramik nach Anspruch 14.

Die erfindungsgemäße Lithiumsilikat-Glaskeramik zeichnet sich dadurch aus, dass sie 8,0 bis 16,0 Gew.-% ZrO₂ und 0,5 bis 3,5 Gew.-% Al₂O₃ enthält
und Lithiummetasilikat als Hauptkristallphase aufweist und in Form eines monolithischen Rohlings vorliegt.

In einer weiteren bevorzugten Ausführungsform enthält die Glaskeramik insbesondere 10,0 bis 16,0 Gew.-% ZrO₂.

Bevorzugt ist weiter eine Glaskeramik, die 55,0 bis 71,0, bevorzugt 60,0 bis 71,0 und insbesondere 60 bis 69 Gew.-% SiO₂ enthält.

Außerdem ist eine Glaskeramik bevorzugt, die 9,0 bis 17,0 und insbesondere 11 bis 15 Gew.-% Li₂O enthält.

Weiter hat es sich als besonders bevorzugt erwiesen, wenn die Glaskeramik 0,5 bis 12,0 und insbesondere 2,5 bis 7,0 Gew.-% Keimbildner enthält. Bevorzugte Keimbildner sind ausgewählt aus P₂O₅, TiO₂, Nb₂O₅, Metallen, z.B. Pt, Pd, Au und Ag, oder Mischungen davon. Besonders bevorzugt enthält die Glaskeramik P₂O₅ als Keimbildner. Überraschenderweise bewirkt insbesondere P₂O₅ als Keimbildner die Ausbildung von gewünschten Lithiumdisilikat-Kristallen und vermeidet auf der anderen Seite weitgehend die Bildung von ZrO₂-haltigen Kristallphasen, die die Transluzenz erheblich verschlechtern könnten. Auch wird durch seine Verwendung offenbar die Ausbildung von anderen unerwünschten Nebenkristallphasen weitgehend vermieden.

Die erfindungsgemäße Glaskeramik enthält bevorzugt weiteres Alkalimetalloxid in einer Menge von 1,0 bis 7,0, bevorzugt 2,0 bis 7,0 und besonders bevorzugt 2,0 bis 5,0 Gew.-%. Der Begriff "weiteres Alkalimetalloxid" bezeichnet Alkalimetalloxid mit Ausnahme von Li₂O. Das weitere Alkalimetalloxid ist insbesondere K₂O, Cs₂O und/oder Rb₂O und ist besonders bevorzugt K₂O. Es wird angenommen, dass der Einsatz von K₂O gegenüber dem in konventionelle Glaskeramiken eingesetzten Na₂O zur Verstärkung des Glasnetzwerkes beiträgt. Es ist bevorzugt, dass die Glaskeramik weniger als 2,0, insbesondere weniger als 1,0, bevorzugt weniger als 0,5 und besonders bevorzugt im Wesentlichen kein Na₂O enthält.

Weiter ist es bevorzugt, dass die Glaskeramik bis zu 5,0 Gew.-% Erdalkalimetalloxid enthält, wobei das Erdalkalimetalloxid insbesondere CaO, BaO, MgO, SrO oder eine Mischung davon ist.

Es ist weiter eine Glaskeramik bevorzugt, die 0,5 bis 10,0, insbesondere 2,5 bis 7,0 und bevorzugt 2,5 bis 3,5 Gew.-% Oxid dreiwertiger Elemente enthält, wobei dieses Oxid insbesondere ausgewählt ist aus Al₂O₃, Y₂O₃, La₂O₃, Bi₂O₃ und Mischungen davon, und bevorzugt Al₂O₃ ist.

Besonders bevorzugt ist eine Glaskeramik, die mindestens eine und bevorzugt alle folgenden Komponenten enthält:

| Komponente | Gew.-% |
|---|---|
| SiO₂ | 55,0 bis 71,0 |
| Li₂O | 9,0 bis 17,0 |
| K₂O | 1,0 bis 7,0, insbesondere 2,0 bis 5,0 |
| Al₂O₃ | 0,5 bis 3,5 |
| P₂O₅ | 0,5 bis 12,0, insbesondere 2,5 bis 7,0 |
| ZrO₂ | 8,0 bis 16,0. |

Die erfindungsgemäße Glaskeramik kann darüber hinaus noch Zusatzkomponenten enthalten, die insbesondere ausgewählt sind aus weiteren Oxiden vierwertiger Elemente, weiteren Oxiden fünfwertiger Elemente, Oxiden sechswertiger Elemente, Schmelzbeschleunigern, Färbemitteln und Fluoreszenzmitteln.

Der Begriff "weitere Oxide vierwertiger Elemente" bezeichnet Oxide vierwertiger Elemente mit Ausnahme von SiO₂ und ZrO₂. Beispiele für weitere Oxide vierwertiger Elemente sind SnO₂ und GeO₂.

Der Begriff "weitere Oxide fünfwertiger Elemente" bezeichnet Oxide fünfwertiger Elemente mit Ausnahme von P₂O₅. Ein Beispiel für ein weiteres Oxid fünfwertiger Elemente ist Bi₂O₅.

Beispiele für Oxide sechswertiger Elemente sind WO₃ und MoO₃.

Bevorzugt ist eine Glaskeramik, die mindestens ein weiteres Oxid vierwertiger Elemente, ein weiteres Oxid fünfwertiger Elemente oder ein Oxid sechswertiger Elemente enthält.

Beispiele für Schmelzbeschleuniger sind Fluoride.

Beispiel für Färbemittel und Fluoreszenzmittel sind Oxide von d- und f-Elementen, wie z.B. die Oxide von Ti, Sc, Mn, Fe, Ag, Ta, W, Ce, Pr, Nd, Tb, Er und Yb.

Der im Folgenden verwendete Begriff "Hauptkristallphase" bezeichnet die Kristallphase, die gegenüber anderen Kristallphasen den höchsten Volumenanteil hat.

Die erfindungsgemäße Glaskeramik weist Lithiummetasilikat als Hauptkristallphase auf. Insbesondere enthält die Glaskeramik mehr als 10 Vol.-%, bevorzugt mehr als 20 Vol.-% und besonders bevorzugt mehr als 30 Vol.-% an Lithiummetasilikat-Kristallen, bezogen auf die gesamte Glaskeramik.

Diese Glaskeramik kann durch Wärmebehandlung in eine Glaskeramik umgewandelt werden, die Lithiumdisilikat als Hauptkristallphase aufweist. Insbesondere enthält die Glaskeramik mehr als 10 Vol.-%, bevorzugt mehr als 20 Vol.-% und besonders bevorzugt mehr als 30 Vol.-% an Lithiumdisilikat-Kristallen, bezogen auf die gesamte Glaskeramik.

Die Lithiumdisilikat-Glaskeramik zeichnet sich durch besonders gute mechanische Eigenschaften aus und sie kann durch Wärmebehandlung der erfindungsgemäßen Lithiummetasilikat-Glaskeramik erzeugt werden.

Es hat sich überraschenderweise gezeigt, dass die Lithiumdisilikat-Glaskeramik trotz ihres hohen Gehalts an ZrO₂ vorteilhafte mechanische Parameter, wie hohe Bruchzähigkeits-Werte hat und insbesondere Aufpressen im viskosen Zustand auf Zirkonoxidkeramik aufgebracht werden kann, ohne dass es dabei zu Verspannungen in der
Glaskeramik kommt, die sich durch Risse oder Sprünge bemerkbar machen. Es ist dabei besonders überraschend, dass diese sehr guten mechanischen Eigenschaften erzielt werden, obwohl das Gefüge der Glaskeramik Lithiumdisilikat-Kristalle aufweist, die in der Regel nicht miteinander vernetzt sind. Eine derartige Vernetzung tritt hingegen bei den bekannten Lithiumdisilikat-Glaskeramiken auf und sie wird als wesentlicher Grund für deren hohe Festigkeiten angesehen. Es wird derzeit angenommen, dass das ZrO₂ in der erfindungsgemäßen Glaskeramik anders als in bekannten Produkten nicht als Keimbildner für andere Kristallphasen dient, sondern vielmehr das Glasnetzwerk über darin eingebaute Zr-O-Polyeder verstärkt. Diese Polyeder können [ZrO_{6/2}]²⁻- oder [ZrO_{8/2}]⁴⁻ Struktureinheiten sein, die als Netzwerkbildner oder Netzwerkwandler fungieren.

Auch ist es überraschend, dass die Lithiumdisilikat-Glaskeramik trotz ihres hohen Gehalts an ZrO₂ eine hohe Transluzenz besitzt und bei ihr keine Amorph-Amorph-Phasentrennung auftritt und sie damit zur ästhetisch ansprechenden Beschichtung von insbesondere dentalen Restaurationen auf Basis von Zirkonoxidkeramik eingesetzt werden kann.

Die in der Lithiumdisilikat-Glaskeramik vorhandenen Lithiumdisilikat-Kristalle haben insbesondere die Form von Plättchen. Es wird angenommen, dass diese spezielle Morphologie den rissfreien Werkstoffverbund mit Zirkonoxidkeramiken ermöglicht. Der kritische Spannungsaufbau im Werkstoffverbund während der thermischen Abkühlphase scheint bei der plättchenförmigen Kristallform weniger stark ausgeprägt zu sein als bei Lithiumdisilikat-Glaskeramiken mit länglichen oder nadelförmigen Kristallen. Zudem wird mit der plättchenförmigen Kristallmorphologie eine gute Bruchzähigkeit, ausgedrückt durch den K_{IC}-Wert, erreicht.

Die Lithiumdisilikat-Glaskeramik hat insbesondere eine Bruchzähigkeit, gemessen als K_{IC} Wert, von mindestens 1.5 MPa•m^{0.5} und insbesondere mehr als 1.8 MPa•m^{0.5}. Weiter hat sie eine hohe biaxiale Bruchfestigkeit von bevorzugt 200 bis 500 MPa. Überdies zeigt sie eine hohe chemische Beständigkeit, die
durch Masseverlust nach Lagerung in Essigsäure ermittelt wurde. Die chemische Beständigkeit beträgt insbesondere weniger als 60 µg/cm². Schließlich hat sie einen linearen thermischen Ausdehnungskoeffizienten von insbesondere weniger als 10,3 x 10⁻⁶ K⁻¹ m/m, gemessen im Bereich von 100 bis 500°C, der damit regelmäßig geringer ist als der der zu beschichtenden Zirkonoxidkeramik.

Durch Wärmebehandlung eines Ausgangsglases, das die Komponenten der erfindungsgemäßen Glaskeramik enthält, kann ein Lithiumsilikatglas mit Keimen erzeugt werden, die zur Ausbildung von Lithiummetasilikatkristallen geeignet sind. Durch eine weitere Wärmebehandlung kann dann die erfindungsgemäße Lithiummetasilikat-Glaskeramik gebildet werden, die ihrerseits durch weitere Wärmebehandlung in die oben beschriebene Lithiumdisilikat-Glaskeramik umgewandelt werden kann. Mithin können das Ausgangsglas, das Glas mit Keimen und die Lithiummetasilikat-Glaskeramik als Vorstufen zur Erzeugung der hochfesten Lithiumdisilikat-Glaskeramik angesehen werden.

Die Erfindung betrifft ebenfalls ein Verfahren zur Herstellung einer Glaskeramik, die 8,0 bis 16,0 Gew.-% ZrO₂ und 0,5 bis 3,5 Gew.-% Al₂O₃ enthält und Lithiummetasilikat als Hauptkristallphase aufweist, bei dem ein Ausgangsglas mit den Komponenten
der Glaskeramik in Form eines Massivglasrohlings mindestens einer Wärmebehandlung im Bereich von 450 bis 950°C unterzogen wird.

Das Ausgangsglas enthält daher 8,0 bis 16,0 Gew.-% ZrO₂ und 0,5 bis 3,5 Gew.-% Al₂O₃. Darüber hinaus enthält es bevorzugt auch geeignete Mengen an SiO₂ und Li₂O, um die Ausbildung einer Lithiumsilikat-Glaskeramik zu ermöglichen. Weiter kann das Ausgangsglas auch noch andere Komponenten enthalten, wie sie oben für die erfindungsgemäße Lithiumsilikat-Glaskeramik angegeben sind. Dabei sind solche Ausführungsformen bevorzugt, die auch für die Glaskeramik als bevorzugt angegeben sind.

Zur Herstellung des Ausgangsglases wird insbesondere so vorgegangen, dass eine Mischung von geeigneten Ausgangsmaterialien, wie z.B. Carbonaten, Oxiden, Phosphaten und Fluoriden, bei Temperaturen von insbesondere 1300 bis 1600°C für 2 bis 10 h erschmolzen wird. Zur Erzielung einer besonders hohen Homogenität wird die erhaltene Glasschmelze in Wasser gegossen, um ein Glasgranulat zu bilden, und das erhaltene Granulat wird dann erneut aufgeschmolzen. Die Schmelze wird dann in Formen gegossen, um Rohlinge des Ausgangsglases, sogenannte Massivglasrohlinge oder monolithische Rohlinge, zu erzeugen.

Anschließend wird das Ausgangsglas in Form eines Massivglasrohlings mindestens einer Wärmebehandlung im Bereich von 450 bis 950°C unterzogen. Es ist bevorzugt, dass zunächst bei einer Temperatur im Bereich von 500 bis 600°C eine erste Wärmebehandlung durchgeführt wird, um ein Glas mit Keimen herzustellen, welche zur Bildung von Lithiummetasilikatkristallen geeignet sind. Dieses Glas kann dann bevorzugt mindestens einer weiteren Temperaturbehandlung bei einer höheren Temperatur und insbesondere mehr als 570°C unterworfen werden, um Kristallisation von Lithiummetasilikat zu bewirken.

Die im erfindungsgemäßen Verfahren durchgeführte mindestens eine Wärmebehandlung kann auch im Rahmen des Aufpressens des erfindungsgemäßen Glases oder der erfindungsgemäßen Glaskeramik auf die ausgewählte Zirkonoxidkeramik erfolgen.

Aus den erfindungsgemäßen Glaskeramiken können dentale Restaurationen, wie Inlays, Onlays, Kronen, Veneers, Schalen oder Abutments, hergestellt werden. Die Erfindung betrifft daher auch deren Verwendung zur Herstellung dentaler Restaurationen. Dabei ist es bevorzugt, dass die Glaskeramik durch Verpressen oder maschinelle Bearbeitung zur gewünschten dentalen Restauration verformt wird. Das Verpressen erfolgt üblicherweise unter erhöhtem Druck und erhöhter Temperatur. Es können für das Verpressen vor allem die erfindungsgemäße Lithiummetasilikat-Glaskeramik in geeigneter Weise in Form von Rohlingen eingesetzt werden. Die maschinelle Bearbeitung wird üblicherweise im Rahmen eines CAD/CAM-Verfahrens durchgeführt, und sie nutzt insbesondere die erfindungsgemäße Lithiummetasilikat-Glaskeramik in Form von geeigneten Rohlingen. Nach der Herstellung der gewünscht geformten dentalen Restauration durch Verpressen oder maschinelle Bearbeitung kann diese insbesondere noch wärmebehandelt werden, um die Lithiummetasilikat-Glaskeramik in Lithiumdisilikat-Glaskeramik umzuwandeln.

Die erfindungsgemäße Glaskeramik eignet sich allerdings insbesondere zur Beschichtung von Zirkonoxidkeramiken. Die Erfindung ist daher ebenfalls auf die Verwendung der erfindungsgemäßen Glaskeramik zur Beschichtung von Zirkonoxidkeramiken gerichtet.

Die Beschichtung von Zirkonoxidkeramik kann durch ein Verfahren erfolgen, bei dem die erfindungsgemäße Glaskeramik auf die Zirkonoxidkeramik aufgebracht und erhöhter Temperatur ausgesetzt wird.

Dies kann insbesondere durch Aufpressen erfolgen. Beim Aufpressen wird die erfindungsgemäße Glaskeramik in Form von monolithischen Rohlingen, bei einer erhöhten Temperatur, von z.B. 700 bis 1200°C, und unter Anwendung von Druck, z.B. 2 bis 10 bar, aufgepresst. Hierzu können insbesondere die in der EP 231 773 beschriebenen Verfahren und der dort offenbarte Pressofen eingesetzt werden. Ein geeigneter Ofen ist z.B. der Programat EP 5000 von Ivoclar Vivadent AG, Liechtenstein.

Es ist bevorzugt, dass nach Abschluss des Beschichtungsvorganges die oben beschriebene Glaskeramik mit Lithiumdisilikat als Hauptkristallphase vorliegt, da sie über besonders gute Eigenschaften verfügt. Es zeigt sich überraschenderweise, dass die Glaskeramik praktisch keine Sprünge und Risse aufweist, nachdem sie auf die Zirkonoxidkeramik geschichtet worden ist, und es wird ein fester Verbund zwischen Glaskeramik und Keramik erzielt.

Es ist bevorzugt, dass die Zirkonoxidkeramik zur Stabilisierung der tetragonalen Phase mindestens ein Oxid des Ce, Y, Sr, Ca oder Mg enthält. Die Zirkonoxidkeramik kann auch in Form eines Komposits mit anderen anorganischen Komponenten vorliegen.

Die mit der erfindungsgemäßen Glaskeramik beschichtete Zirkonoxidkeramik stellt einen weiteren Gegenstand der Erfindung dar.

Aufgrund der vorstehend geschilderten Eigenschaften der erfindungsgemäßen Glaskeramik eignet sich diese insbesondere auch zum Einsatz in der Zahnheilkunde. Gegenstand der Erfindung ist daher auch die Verwendung der erfindungsgemäßen Glaskeramik als Dentalmaterial und insbesondere zur Herstellung dentaler Restaurationen oder als Beschichtungsmaterial für dentale Restaurationen, wie Kronen und Brücken.

Es ist überraschend, dass im Verbund zwischen der oben beschriebenen Lithiumdisilikat-Glaskeramik und Zirkonoxidkeramik keine Risse in der Glaskeramik auftreten. Es wird vermutet, dass insbesondere die spezielle plättchenförmige Morphologie der Lithiumdisilikatkristalle hierfür von Bedeutung ist. Der kritische Spannungsaufbau im Werkstoffverbund während der thermischen Abkühlphase scheint bei der plättchenförmigen Kristallform weniger stark ausgeprägt zu sein als bei Lithiumdisilikat-Glaskeramiken mit länglichen oder nadelförmigen Kristallen. Zudem wird insbesondere mit der plättchenartigen Kristallmorphologie eine gute Bruchzähigkeit von bis zu 2,1 MPa·m^{0,5} erreicht, obwohl im Gefüge im wesentlichen keine direkte Vernetzung der Lithiumdisilikat-Kristalle erkennbar ist. Damit ist die beschichtete Zirkonoxidkeramik ein starker Verbund aus einerseits hochfester und hochzäher Zirkonoxidkeramik und andererseits zäher Glaskeramik, weshalb dieser Verbund zu hohen Lastaufnahmen im Kauzyklus in der Lage ist. Damit kann die erfindungsgemäße Glaskeramik vorteilhaft auch gerade bei der Beschichtung von langspannigen Brücken mit mehr als drei Gliedern auf Basis von Zirkonoxidkeramik verwendet werden.

Die Erfindung wird im Folgenden anhand von Beispielen näher erläutert.

### Beispiele

### Beispiele 1 bis 20 - Zusammensetzung und Kristallphasen

Es wurden insgesamt 20 Gläser und Glaskeramiken mit der aus den Tabellen I bis IV angegebenen Zusammensetzung über Erschmelzung entsprechender Ausgangsgläser und anschließende Wärmebehandlung zur gesteuerten Keimbildung und Kristallisation hergestellt.

Dazu wurden zunächst die Ausgangsgläser im 100 bis 200 g Massstab aus üblichen Rohstoffen bei 1400 bis 1500°C erschmolzen und durch Eingießen in Wasser in Glasfritten umgewandelt. Diese Glasfritten wurden zur Homogenisierung anschließend ein zweites Mal bei 1450 bis 1550 °C für 1 bis 3 h geschmolzen. Die erhaltenen Glasschmelzen wurden in vorgewärmte Formen gegossen, um Glasmonolithe zu erzeugen. Diese Glasmonolithe wurden durch thermische Behandlung zu Gläsern und Glaskeramiken umgewandelt.

Die angewandte thermische Behandlung zur gesteuerten Keimbildung und gesteuerten Kristallisation ist in der Tabelle V für ausgewählte Beispiele angegeben. Dabei führte in der Regel die erste Wärmebehandlung im Bereich von 500 bis 560°C zur Bildung von Lithiumsilikat-Gläsern mit Keimen für Lithiummetasilikat- oder Lithiumdisilikatkristalle, die zweite Wärmebehandlung bei 650 bis 710°C zur Bildung von Lithiummetasilikat-Glaskeramiken und die dritte Wärmebehandlung im Bereich von 800 bis 920°C zur Bildung von Lithiumdisilikat-Glaskeramiken.

Bei einigen Beispielen wurde nach einer ersten Wärmebehandlung eine zweite nicht-isotherme Wärmebehandlung mit gleichzeitiger Analyse der gebildeten Kristallphasen bei der jeweils angegebenen Temperatur durch Hochtemperatur-Röntgenbeugung (HT-XRD) durchgeführt.

Die nach Abschluß aller Wärmebehandlungen erhaltenen Kristallphasen sind ebenfalls in Tabelle V aufgeführt. Es wurden überraschenderweise stets Glaskeramiken mit Lithiumdisilikat als Hauptkristallphase erhalten. Die Beispiele 4 und 5 wurden zusätzlich wiederholt, indem lediglich die erste und zweite Wärmebehandlung durchgeführt wurden. Auf diese Weise wurden Glaskeramiken mit Lithiummetasilikat als Hauptkristallphase erzeugt.

### Beispiel 21 - Glas- und Glaskeramikrohlinge

Es wurde ein Glas mit der Zusammensetzung gemäß Beispiel 4 hergestellt, indem entsprechende Rohstoffe in Form von Oxiden und Carbonaten 30 min in einem Turbola-Mischer gemischt und anschliessend bei 1450°C für 120 min in einem Platintiegel erschmolzen wurden. Die Schmelze wurde in Wasser gegossen, um ein feinteiliges Glasgranulat zu erhalten. Dieses Glasgranulat wurde erneut bei 1530°C für 150 min geschmolzen, um eine Glasschmelze mit besonders hoher Homogenität zu erhalten. Die Temperatur wurde für 30 min auf 1500°C abgesenkt und anschließend wurden zylindrische Glasrohlinge mit einem Durchmesser von 12.5 mm in vorgeheizte, teilbare Stahlformen oder Graphitformen gegossen. Danach wurden die erhaltenen Glaszylinder bei 550°C entspannt. Es wurde ein Glas mit Keimen für Lithiummetasilikat- oder Lithiumdisilikat-Kristalle erhalten.

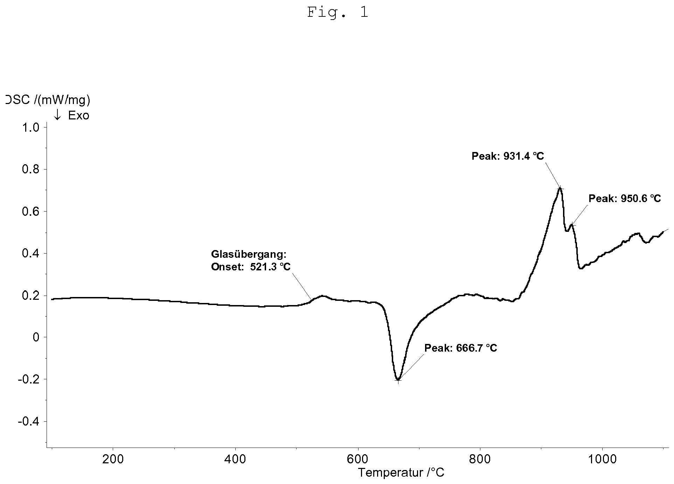

Die Figur 1 zeigt das Ergebnis der Differentialthermoanalyse (DSC) eines zerstossenen Glaszylinders.

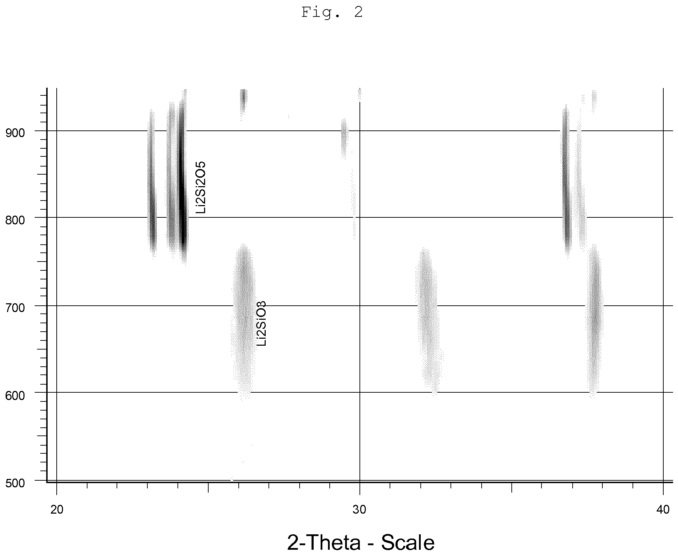

Die Figur 2 zeigt anhand von Hochtemperatur-Röntgenbeugung (HT-XRD) eines Glaszylinders die Abhängigkeit der Bildung von Lithiummetasilikat (Li2SiO3) und Lithiumdisilikat (Li2Si2O5) von der Temperatur.

Die Glaszylinder wurden dann einer ersten Kristallisation bei 680 bis 700°C für 20 min unterworfen. Dabei betrug die Aufheizgeschwindigkeit 15°C pro Minute. Die Glaszylinder wurden anschließend einer zweiten Kristallisation bei 850 bis 880°C für 30 min unterworfen. Die Kristallphasenanalyse zeigte nach dieser Behandlung eine Glaskeramik mit Lithiumdisilikat als Hauptkristallphase sowie geringen Anteilen Lithiummetasilikat und Lithiumphosphat als Nebenphasen an.

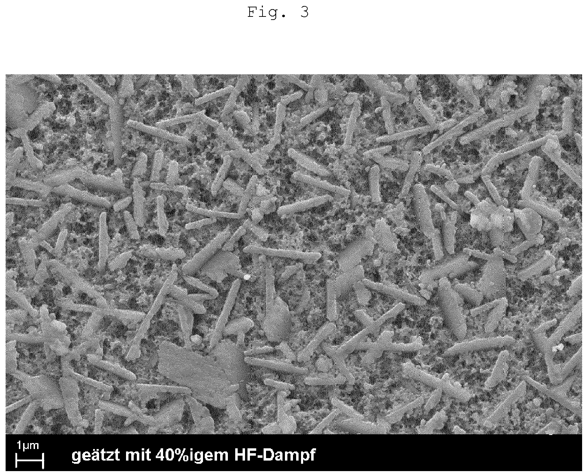

Die Figur 3 zeigt eine rasterelektronenmikroskopische (REM) Aufnahme eines kristallisierten Zylinders, der poliert und 30 s mit HF-Dampf geätzt worden ist.

Die kristallisierten Zylinder wurden außerdem durch Heißpressen bei einer Presstemperatur von 910°C unter Verwendung eines Pressofens EP600, Ivoclar Vivadent AG, zu Prüfkörpern weiterverarbeitet. Die Eigenschaften dieser Prüfkörper waren wie folgt:

| | |
|---|---|
| Farbe: | weiss transluzent ohne Fluoreszenz |
| Löslichkeit: | 24 µg/cm² (nach ISO 6872 vom 1. September 2008) |
| Biaxialfestigkeit: | 420 MPa (nach ISO 6872 vom 1. September 2008) |
| Bruchzähigkeit: | 2,0 MPam^{0,5} (bestimmt als K_{IC}-Wert nach der SEVNB-Methode gemäß ISO 6872 vom 1. September 2008) |
| Thermischer Ausdehnungskoeffizient: | 9.9*10⁻⁶*1/K (im Bereich 100 bis 500°C) |

### Beispiel 22 - Heisspressen auf Zirkonoxidkeramik

Durch Heisspressen wurde die Lithiumdisilikat-Glaskeramik gemäß Beispiel 4 bei 920°C in einem kombinierten Press- und Brennofen Programat EP 5000 der Firma Ivoclar Vivadent AG, Liechtenstein, auf Zirkonoxidkeramik vom Typ 3 Y-TZP, erhältlich von der Firma Tosoh, aufgepresst. Nach Abschluss des Beschichtungsvorganges ergab sich eine defektfreie Fügung.

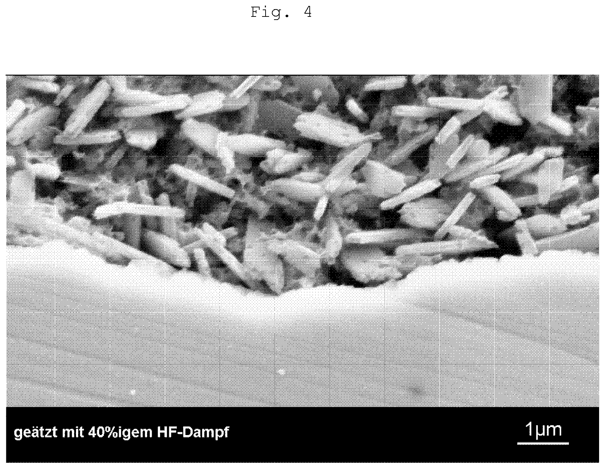

Figur 4 zeigt eine rasterelektronenmikroskopische Aufnahme (REM) dieses Verbundes nach Ätzen mit 40%-igem HF-Dampf.

### Beispiel 23 - Heisspressen auf Zahnkäppchen und Brückengerüste

Einzelzahnkäppchen und viergliedrige Brückengerüste aus dicht gesintertem Zirkonoxid (e.max ZirCAD, Ivoclar Vivadent AG) wurden mit einem ausbrennbaren Kunststoff (PMMA) zu anatomisch geformten Restaurationen ergänzt. Sowohl die Brückengerüste als auch die Kunststoffteile wurden mittels CAD/CAM-Verfahren gefertigt, wodurch eine reproduzierbare Geometrie und Schichtdicke erzielt werden konnte. Die Restaurationen wurden in dentaler Einbettmasse (IPS PressVest Speed, Ivoclar Vivadent AG) eingebettet, der Kunststoff wurde ausgebrannt und die kristallisierten Zylinder gemäß Beispiel 21 wurden bei einer Temperatur von 910 °C direkt auf die Gerüste aufgepresst. Es wurde keine Zwischenschicht (Liner) auf das Zirkonoxid aufgebracht.

Nach dem vollständigen Abkühlen wurden die Objekte mit einem Sandstrahlgerät ausgebettet, wobei aufgrund der hohen Festigkeit der aufgeschichteten Glaskeramik keine besondere Vorsicht erforderlich war. Die Objekte wurden von den Presskanälen abgetrennt, mit einem Diamantschleifer trocken überarbeitet, und dann 20 min mit IPS INVEX Liquid (Ivoclar Vivadent AG) unter Ultraschall behandelt, um noch verbliebene Reste an Einbettmasse zu lösen, welche dann mit Al₂O₃-Sand der Korngrösse 100 µm bei 1-2 bar Druck abgestrahlt wurden.

Die Oberfläche wurden mit Heissdampf gereinigt und mit IPS e.max Ceram Glaze (Ivoclar Vivadent AG) bei 770°C zweimal glasiert, wodurch ein schöner Glanz erzeugt wurde. Bei den Glasurbränden wurde keine spezielle Abkühlung (Entspannungskühlung) angewandt. Die so hergestellten Restaurationen, d.h. Kronen und Brücken, waren ästhetisch ansprechend und fehlerfrei. Sie zeigten keine Risse, Blasen oder Abhebungen. Nach Aufsägen war im Querschnitt ein hervorragender Verbund zwischen der aufgeschichteten Lithiumdisilikat-Glaskeramik und dem Zirkonoxid mittels REM zu erkennen.

Jeweils 8 Kronen und 8 Brücken wurden in einer Kaumaschine (Willitec) mit 300.000 Zyklen bei Wasserlagerung unter Thermocycling von 5 auf 55 °C belastet. Die Prüfkraft betrug dabei während je 100.000 Zyklen 30, 60 bzw. 90 N. Die Belastung wurde mit einer Frequenz von 0.8 Hz aufgebracht. Hierbei zeigten sich keinerlei Abplatzungen (Chippings) in der Verblendstruktur.

### Beispiel 24 - Glas- und Glaskeramikrohlinge

Es wurde das Beispiel 21 mit dem Unterschied wiederholt, dass von einem Glas mit der Zusammensetzung gemäß Beispiel 15 ausgegangen wurde. Die erhaltenen kristallisierten Zylinder wurden durch Heisspressen bei einer Temperatur von 905°C zu Prüfkörpern weiterverarbeitet. Die Eigenschaften dieser Prüfkörper waren wie folgt:

| | |
|---|---|
| Farbe: | zahnfarben transluzent mit zahnähnlicher Fluoreszenz |
| Löslichkeit: | 30 µg/cm² (nach ISO 6872 vom 1. September 2008) |
| Biaxialfestigkeit: | 405 MPa (nach ISO 6872 vom 1. September 2008) |
| Thermischer Ausdehnungskoeffizient: | 9.9*10⁻⁶*1/K (im Bereich 100 bis 500°C) |

### Beispiel 25 - Heisspressen auf Zahnkäppchen und Brückengerüste

Es wurde das Beispiel 23 mit dem Unterschied wiederholt, dass die kristallisierten Zylinder gemäß Beispiel 24 eingesetzt wurden. Nach bis zu vier abschließenden Glasurbränden wurden Kronen und Brücken erhalten, die wiederum keine Risse, Blasen oder Abhebungen zeigten.

### Beispiel 26 - Direkte Herstellung dentaler Restaurationen durch Heißpressen oder maschinelle Bearbeitung (CAD/CAM)

### (A) Glasrohlinge mit Keimen

Es wurden zunächst Gläser mit der Zusammensetzung gemäß Beispielen 2, 3 und 4 hergestellt, indem entsprechende Rohstoffe in Form von Oxiden und Carbonaten 30 min in einem Turbola-Mischer gemischt und anschließend bei 1450°C für 120 min in einem Platintiegel erschmolzen wurden. Die Schmelzen wurden in Wasser gegossen, um feinteilige Glasgranulate zu erhalten. Diese Glasgranulate wurden erneut bei 1530°C für 150 min geschmolzen, um Glasschmelzen mit besonders hoher Homogenität zu erhalten. Die Temperatur wurde für 30 min auf 1500°C abgesenkt und anschließend wurden a) quaderförmige (12.5mm x 14mm x 40mm) und b) zylindrische Glasrohlinge mit einem Durchmesser von 12.5mm in vorgeheizte, teilbare Stahlformen oder Graphitformen gegossen. Danach wurden die erhaltenen Glasquader oder Glaszylinder im Bereich von 500-560°C je nach Zusammensetzung wärmebehandelt, um Keime für Lithiummetasilikat- und/oder Lithiumdisilikat-Kristalle auszubilden und das Glas zu entspannen.

Die erhaltenen Rohlinge mit Keimen wurden gemäß der folgenden Alternativen zu Restaurationen weiterverarbeitet.

### (B) Heißpressen von Glas mit Keimen, Lithiummetasilikat- oder Lithiumdisilikat-Glaskeramik

i) Die Glaszylinder mit Keimen (A) wurden durch Heißpressen bei einer Presstemperatur von 900-950°C unter Verwendung eines Pressofens EP600, Ivoclar Vivadent AG, zu dentalen Restauration, wie Inlays, Onlays, Veneers, Teilkronen, Kronen und Schalen, verarbeitet. Als Hauptkristallphase konnte in den Restaurationen Lithiumdisilikat nachgewiesen werden.
ii) Die Glaszylinder mit Keimen (A) wurden einer ersten Kristallisation bei 650 bis 750°C für 20 min unterworfen. Dabei betrug die Aufheizgeschwindigkeit 15°C pro Minute. Nach dieser ersten Kristallisation konnte als Hauptkristallphase Lithiummetasilikat nachgewiesen werden. Durch Heißpressen der Lithiummetasilikat-Glaszylinder bei einer Presstemperatur von 900-950°C unter Verwendung eines Pressofens EP600, Ivoclar Vivadent AG, konnten dentale Restaurationen, wie Inlays, Onlays, Veneers, Teilkronen, Kronen und Schalen, hergestellt werden. Dabei wurde durch das Heißpressen Lithiummetasilikat in Lithiumdisilikat umgewandelt.
iii) Die Glaszylinder mit Keimen (A) wurden nach einer ersten Kristallisation gemäß ii) zusätzlich einer weiteren thermischen Behandlung bei 840 bis 880°C für 5 bis 30 min. unterworfen. Die Kristallphasenanalyse zeigte nach dieser Behandlung eine Glaskeramik mit Lithiumdisilikat als Hauptkristallphase. Die nach der zweiten Kristallisation erhaltenen kristallisierten Zylinder wurden anschließend durch Heißpressen bei einer Presstemperatur von 900-950°C unter Verwendung eines Pressofens EP600, Ivoclar Vivadent AG, zu dentalen Restaurationen, wie Inlays, Onlays, Veneers, Kronen, Teilkronen und Schalen, weiterverarbeitet.

### (C) Maschinelle Bearbeitung (CAD/CAM-Verfahren) von Lithiummetasilikat

Die Glasquader mit Keimen (A) wurden zunächst einer ersten Kristallisation gemäß (B)(ii) unterzogen, um die Kristallisation von Lithiummetasilikat zu bewirken. Aus den erhaltenen Lithiummetasilikat-Glasquadern wurden dann mittels CAD/CAM-Verfahren, z.B. Sirona, Cerec 2® oder Cerec 3®, maschinell dentale Restaurationen, wie Inlays, Onlays, Kronen, Teilkronen, Schalen und Veeners, herausgearbeitet. Anschließend wurden diese Restaurationen einer zweiten Kristallisation bei 840 bis 880°C für 5 min bis 1 h unterworfen. Die Kristallphasenanalyse zeigte nach dieser Behandlung eine Glaskeramik mit Lithiumdisilikat als Hauptkristallphase.

**Tabelle I**

| | 1 | | 2 | 3 | 4 | 5 | 6 | | |
|---|---|---|---|---|---|---|---|---|---|
| **SiO₂** | 63.8 | | 67.9 | 66.4 | 65.0 | 63.5 | 62.0 | | |
| **K₂O** | 3.0 | | 3.7 | 3.6 | 3.5 | 3.4 | 3.4 | | |
| **Li₂O** | 13.6 | | 14.1 | 13.8 | 13.5 | 13.2 | 12.9 | | |
| **Al₂O₃** | 3.0 | | 3.2 | 3.2 | 3.1 | 3.0 | 2.9 | | |
| **P₂O₅** | 3.0 | | 3.1 | 3.0 | 2.9 | 2.9 | 2.8 | | |
| **ZrO₂** | 9.6 | | 8.0 | 10.0 | 12.0 | 14.0 | 16.0 | | |
| **MoO₃** | 4.0 | | | | | | | | |
| | 100.0 | | 100.0 | 100.0 | 100.0 | 100.0 | 100.0 | | |

**Tabelle II**

| | | | 7 | 8 | | | 9 | | 10 | 11 | 12 |
|---|---|---|---|---|---|---|---|---|---|---|---|
| **SiO₂** | | | 65.2 | 60.5 | | | 66.4 | | 70.1 | 64.3 | 64.2 |
| **K₂O** | | | 3.5 | 3.3 | | | | | 3.6 | 3.0 | 1.0 |
| **Li₂O** | | | 12.0 | 15.0 | | | 13.6 | | 9.0 | 13.2 | 13.2 |
| **Na₂O** | | | | | | | | | 0.1 | | |
| **CaO** | | | | | | | | | | | |
| **MgO** | | | | | | | | | 0.1 | | |
| **SrO** | | | | | | | | | 0.1 | | 1.0 |
| **Al₂O₃** | | | 3.1 | 2.9 | | | 3.0 | | 3.5 | 2.9 | 0.5 |
| **La₂O₃** | | | | | | | | | | | |
| **Y₂O₃** | | | | | | | | | | | 6.5 |
| **P₂O₅** | | | 4.1 | 5.0 | | | 3.0 | | 3.5 | 2.9 | 3.5 |
| **ZrO₂** | | | 12.1 | 13.3 | | | 10.0 | | 10.0 | 9.7 | 10.1 |
| **Cs₂O** | | | | | | | 4.0 | | | 4.0 | |
| **VO₂** | | | | | | | | | | | |
| | | | 100.0 | 100.0 | | | 100.0 | | 100.0 | 100.0 | 100.0 |

**Tabelle III**

| | 13 | 14 | 15 | 16 |
|---|---|---|---|---|
| **SiO₂** | 61.3 | 62.6 | 64.9 | 64.9 |
| **K₂O** | 3.3 | 5.0 | 3.5 | 3.5 |
| **Li₂O** | 12.7 | 12.7 | 13.5 | 13.5 |
| **CaO** | 3.0 | | | |
| **Al₂O₃** | 2.9 | 2.9 | 3.1 | 3.1 |
| **P₂O₅** | 7.0 | 3.5 | 3.0 | 3.0 |
| **ZrO₂** | 9.0 | 11.3 | 10.4 | 10.9 |
| **F** | 0.5 | | | |
| **MnO₂** | 0.2 | | | |
| **Fe₂O₃** | 0.1 | | | |
| **V₂O₅** | | | 0.1 | |
| **Tb₄O₇** | | 0.4 | 0.5 | 0.5 |
| **CeO2** | | 1.3 | 1.0 | 0.6 |
| **Er₂O₃** | | 0.3 | | |
| | 100.0 | 100.0 | 100.0 | 100.0 |

**Tabelle IV**

| | 17 | 18 | 19 | 20 |
|---|---|---|---|---|
| **SiO₂** | 66.4 | 63.8 | 64.5 | 63.8 |
| **K₂O** | | 3.0 | 3.2 | 3.0 |
| **Li₂O** | 13.6 | 13.6 | 13.8 | 13.6 |
| **Rb₂O** | 4.0 | | | |
| **BaO** | | | 2.0 | |
| **Al₂O₃** | 3.0 | 3.0 | 3.0 | 3.0 |
| **Bi₂O₃** | | | | 4.0 |
| **P₂O₅** | 3.0 | 3.0 | 3.5 | 3.0 |
| **ZrO₂** | 10.0 | 9.6 | 10.0 | 9.6 |
| **WO₃** | | 4.0 | | |
| | 100.0 | 100.0 | 100.0 | 100.0 |

**Tabelle V**

| **Glaskeramik, Nr.** | **Thermische Behandlung (°C/min) oder HT-XRD** | **Kristallphasen HP=Hauptphase NP=Nebenphase(n)** |
|---|---|---|
| | | |
| 2 | 500/10, 650/20, 840/7 | HP:Li₂Si₂O₅ |
| | | NP:Li₃PO₄, Li₂SiO₃ |
| 3 | 500/10, 650/20, 840/7 | HP:Li₂Si₂O₅ |
| | | NP:Li₃PO₄, Li₂SiO₃; Li₄SiO₄ |
| 4 | 540/10, 690/20 | HP:Li₂SiO₃ |
| | | NP:keine |
| 4 | 540/10, 650/20, 840/7 | HP:Li₂Si₂O₅ |
| | | NP:Li₂SiO₃; Li₄SiO₄ |
| 5 | 540/10, 710/20 | HP:Li₂SiO₃ |
| | | NP:keine |
| 5 | 540/10, 650/20, 840/7 | HP:Li₂Si₂O₅ |
| | | NP:Li₄SiO₄ |
| 6 | 560/10 und HT-XRD mit Ausschnitt bei 860 | HP:Li₂Si₂O₅ |
| | | NP:Li₃PO₄, Li₂SiO₃ |
| | | |
| | | |
| | | |
| | | |
| 9 | 520/10, 650/20, 800/10 | HP:Li₂Si₂O₅ |
| | | NP:Li₃PO₄ |
| | | |
| 17 | 520/10, 650/20, 800/10 | HP:Li₂Si₂O₅ |
| | | NP:Li₃PO₄ |
| 18 | 520/10, 650/20, 850/10 | HP: Li₂Si₂O₅ |
| | | NP:Li₃PO₄, Li₂SiO₃ |

Bei der HT-XRD Analyse wurde eine Aufheizgeschwindigkeit von ca. 2 K/min verwendet.

## Patentansprüche

1. Lithiumsilikat-Glaskeramik, die 8,0 bis 16,0 Gew.-% ZrO₂ und 0,5 bis 3,5 Gew.-% Al₂O₃ enthält und Lithiummetasilikat als Hauptkristallphase aufweist und in Form eines monolithischen Rohlings vorliegt.

2. Glaskeramik nach Anspruch 1, die mehr als 10 Vol.-%, bevorzugt mehr als 20 Vol.-% und besonders bevorzugt mehr als 30 Vol.-% an Lithiummetasilikat-Kristallen aufweist.

3. Glaskeramik nach Anspruch 1 oder 2, die 55,0 bis 71,0 und insbesondere 55,0 bis 69,0 Gew.-% SiO₂ enthält.

4. Glaskeramik nach einem der Ansprüche 1 bis 3, die 0,5 bis 12,0 und insbesondere 2,5 bis 7,0 Gew.-% Keimbildner enthält, wobei der Keimbildner insbesondere ausgewählt ist aus P₂O₅, TiO₂, Nb₂O₅ und/oder Metallen und bevorzugt P₂O₅ ist.

5. Glaskeramik nach einem der Ansprüche 1 bis 4, die außer Li₂O weiteres Alkalimetalloxid in einer Menge von 1,0 bis 7,0, bevorzugt 2,0 bis 7,0 und besonders bevorzugt 2,0 bis 5,0 Gew.-% enthält, wobei das weitere Alkalimetalloxid insbesondere K₂O, Cs₂O und/oder Rb₂O ist und bevorzugt K₂O ist.

6. Glaskeramik nach einem der Ansprüche 1 bis 5, die bis zu 5,0 Gew.-% Erdalkalimetalloxid enthält, wobei das Erdalkalimetalloxid bevorzugt CaO, BaO, MgO und/oder SrO ist.

7. Glaskeramik nach einem der Ansprüche 1 bis 6, die 0,5 bis 10,0, insbesondere 2,5 bis 7,0 und bevorzugt 2,5 bis 3,5 Gew.-% Oxid dreiwertiger Elemente enthält.

8. Glaskeramik nach einem der Ansprüche 1 bis 7, die mindestens ein weiteres Oxid vierwertiger Elemente, insbesondere SnO₂ oder GeO₂, ein weiteres Oxid fünfwertiger Elemente, insbesondere Bi₂O₅, oder ein Oxid sechswertiger Elemente, insbesondere WO₃ oder MoO₃, enthält.

9. Glaskeramik nach einem der Ansprüche 1 bis 8, die mindestens eine und bevorzugt alle folgenden Komponenten enthält:
| Komponente | Gew.-% |
|---|---|
| SiO₂ | 55,0 bis 69,0 |
| K₂O | 1,0 bis 5,0 |
| P₂O₅ | 0,5 bis 12,0, insbesondere 2,5 bis 7,0. |

10. Verfahren zur Herstellung einer Lithiumsilikat-Glaskeramik, die 8,0 bis 16,0 Gew.-% ZrO₂ und 0,5 bis 3,5 Gew.-% Al₂O₃ enthält und Lithiummetasilikat als Hauptkristallphase aufweist, bei dem ein Ausgangsglas mit den Komponenten der Glaskeramik in Form eines Massivglasrohlings mindestens einer Wärmebehandlung im Bereich von 450 bis 950°C unterzogen wird.

11. Verwendung der Glaskeramik nach einem der Ansprüche 1 bis 9 als Dentalmaterial und insbesondere zur Beschichtung dentaler Restaurationen und zur Herstellung dentaler Restaurationen.

12. Verwendung nach Anspruch 11 zur Herstellung dentaler Inlays, Onlays, Veneers, Teilkronen, Kronen, Schalen oder Abutments.

13. Dentale Restauration, die mit der Glaskeramik nach einem der Ansprüche 1 bis 9 beschichtet ist.

14. Zirkonoxidkeramik, die mit der Glaskeramik nach einem der Ansprüche 1 bis 9 beschichtet ist.

## Claims

1. Lithium silicate glass ceramic, which comprises 8.0 to 16.0 wt.-% ZrO₂ and 0.5 to 3.5 wt.-% Al₂O₃ and comprises lithium metasilicate as main crystalline phase and is present in the form of a monolithic blank.

2. Glass ceramic according to claim 1, which comprises more than 10 vol.-%, preferably more than 20 vol.-% and particularly preferably more than 30 vol.-% of lithium metasilicate crystals.

3. Glass ceramic according to claim 1 or 2, which comprises 55.0 to 71.0 and in particular 55.0 to 69.0 wt.-% SiO₂.

4. Glass ceramic according to any one of claims 1 to 3, which comprises 0.5 to 12.0 and in particular 2.5 to 7.0 wt.-% nucleating agent, wherein the nucleating agent is selected in particular from P₂O₅, TiO₂, Nb₂O₅ and/or metals and preferably is P₂O₅.

5. Glass ceramic according to any one of claims 1 to 4, which, apart from Li₂O, comprises further alkali metal oxide in an amount of 1.0 to 7.0, preferably 2.0 to 7.0 and particularly preferably 2.0 to 5.0 wt.-%, wherein the further alkali metal oxide is in particular K₂O, Cs₂O and/or Rb₂O and preferably is K₂O.

6. Glass ceramic according to any one of claims 1 to 5, which comprises up to 5.0 wt.-% alkaline earth metal oxide, wherein the alkaline earth metal oxide is preferably CaO, BaO, MgO and/or SrO.

7. Glass ceramic according to any one of claims 1 to 6, which comprises 0.5 to 10.0, in particular 2.5 to 7.0 and preferably 2.5 to 3.5 wt.-% oxide of trivalent elements.

8. Glass ceramic according to any one of claims 1 to 7, which comprises at least one further oxide of tetravalent elements, in particular SnO₂ or GeO₂, one further oxide of pentavalent elements, in particular Bi₂O₅, or one oxide of hexavalent elements, in particular WO₃ or MoO₃.

9. Glass ceramic according to any one of claims 1 to 8, which comprises at least one and preferably all of the following components:
| Component | wt.-% |
|---|---|
| SiO₂ | 55.0 to 69.0 |
| K₂O | 1.0 to 5.0 |
| P₂O₅ | 0.5 to 12.0, in particular 2.5 to 7.0. |

10. Process for preparing a lithium silicate glass ceramic, which comprises 8.0 to 16.0 wt.-% ZrO₂ and 0.5 to 3.5 wt.-% Al₂O₃ and comprises lithium metasilicate as main crystalline phase, in which a starting glass with the components of the glass ceramic in the form of a solid glass blank is subjected to at least one heat treatment in the range of from 450 to 950°C.

11. Use of the glass ceramic according to any one of claims 1 to 9 as a dental material and in particular for coating dental restorations and for preparing dental restorations.

12. Use according to claim 11 for preparing dental inlays, onlays, veneers, partial crowns, crowns, facets or abutments.

13. Dental restoration, which is coated with the glass ceramic according to any one of claims 1 to 9.

14. Zirconium oxide ceramic, which is coated with the glass ceramic according to any one of claims 1 to 9.

## Revendications

1. Vitrocéramique de silicate de lithium, qui contient de 8,0 à 16,0 % en poids de ZrO₂ et de 0,5 à 3,5 % en poids d'Al₂O₃, comporte du métasilicate de lithium en tant que principale phase cristalline, et se présente sous forme de masse de matériau monolithique.

2. Vitrocéramique conforme à la revendication 1, qui comporte plus de 10 % en volume, de préférence plus de 20 % en volume et mieux encore plus de 30 % en volume de cristaux de métasilicate de lithium.

3. Vitrocéramique conforme à la revendication 1 ou 2, qui contient de 55,0 à 71,0 % en poids et en particulier de 55,0 à 69,0 % en poids de SiO₂.

4. Vitrocéramique conforme à l'une des revendications 1 à 3, qui contient de 0,5 à 12,0 % en poids et en particulier de 2,5 à 7,0 % en poids d'agent de formation de germes, lequel agent de formation de germes est choisi en particulier parmi P₂O₅, TiO₂, Nb₂O₅ et/ou les métaux, mais est de préférence du P₂O₅.

5. Vitrocéramique conforme à l'une des revendications 1 à 4, qui, outre du Li₂O, contient un autre oxyde de métal alcalin, en une proportion de 1,0 à 7,0 % en poids, de préférence de 2,0 à 7,0 % en poids et mieux encore de 2,0 à 5,0 % en poids, lequel autre oxyde de métal alcalin est en particulier du K₂O, du Cs₂O et/ou du Rb₂O, mais est de préférence du K₂O.

6. Vitrocéramique conforme à l'une des revendications 1 à 5, qui contient jusqu'à 5,0 % en poids d'un oxyde de métal alcalino-terreux, lequel oxyde de métal alcalino-terreux est de préférence du CaO, du BaO, du MgO et/ou du SrO.

7. Vitrocéramique conforme à l'une des revendications 1 à 6, qui contient de 0,5 à 10,0 % en poids, en particulier de 2,5 à 7,0 % en poids et de préférence de 2,5 à 3,5 % en poids d'oxydes d'éléments trivalents.

8. Vitrocéramique conforme à l'une des revendications 1 à 7, qui contient au moins un autre oxyde d'élément tétravalent, en particulier du SnO₂ ou du GeO₂, un autre oxyde d'élément pentavalent, en particulier du Bi₂O₅, ou un oxyde d'élément hexavalent, en particulier du WO₃ ou du MoO₃.

9. Vitrocéramique conforme à l'une des revendications 1 à 8, qui contient au moins l'un des composants suivants, et de préférence les contient tous :
| Composant | % en poids |
|---|---|
| SiO₂ | 55,0 à 69,0 |
| K₂O | 1,0 à 5,0 |
| P₂O₅ | 0,5 à 12,0 et en particulier 2,5 à 7,0. |

10. Procédé de préparation d'une vitrocéramique de silicate de lithium qui contient de 8,0 à 16,0 % en poids de ZrO₂ et de 0,5 à 3,5 % en poids d'Al₂O₃ et comporte du métasilicate de lithium en tant que principale phase cristalline, dans lequel procédé on soumet un verre de départ, comportant les composants de la vitrocéramique sous forme d'une masse de matériau vitreux massif, au moins à un traitement thermique dans l'intervalle allant de 450 à 950 °C.

11. Utilisation d'une vitrocéramique conforme à l'une des revendications 1 à 9 en tant que matériau dentaire, en particulier pour le revêtement de restaurations dentaires et pour la préparation de restaurations dentaires.

12. Utilisation conforme à la revendication 11, pour la préparation d'incrustations dentaires « inlay » ou « onlay », de facettes « veneer », de couronnes partielles, de couronnes, de coquilles ou d'aboutements.

13. Restauration dentaire qui est revêtue d'une vitrocéramique conforme à l'une des revendications 1 à 9.

14. Céramique de zircone qui est revêtue d'une vitrocéramique conforme à l'une des revendications 1 à 9.
